# EUROPÄISCHE PATENTSCHRIFT

(11) **EP 2 638 919 B1**
(45) Veröffentlichungstag und Bekanntmachung des Hinweises auf die Patenterteilung: **20.07.2016**
(21) Anmeldenummer: 12159092.1
(22) Anmeldetag: 12.03.2012
(51) Int. Cl.: A61L 9/14, A61L 9/015

(54) **Vorrichtung und Verfahren zum Einleiten eines Aromas in einen Trägergasstrom**
Device and method for introducing an aroma into a carrier gas flow
Dispositif et procédé d'introduction d'un arome dans un flux de gaz porteur

(43) Veröffentlichungstag der Anmeldung: 18.09.2013
(73) Patentinhaber: Air Liquide Deutschland GmbH, 40235 Düsseldorf (DE); L'AIR LIQUIDE, Société Anonyme pour l'Etude et l'Exploitation des Procédés Georges Claude, 75007 Paris (FR)
(72) Erfinder: Glogau, Britta, 34260 Kaufungen (DE)
(74) Vertreter: Heine, Christian Klaus

(56) Entgegenhaltungen:
- EP-A1- 0 613 617
- EP-A1- 2 143 779
- WO-A2-2008/004089
- DE-U1-202010 005 686

## Beschreibung

Die vorliegende Erfindung betrifft eine Vorrichtung und ein Verfahren zum Einleiten eines Aromas in einen Trägergasstrom.

EP2143779 offenbart ein Verfahren zum kontrollierten Zumischen eines Odoriermediums zu einem Medium wobei das Odoriermedium und das Medium in einem bzgl. seines Volumens veränderbaren Dosierbehälter vermischt werden.

Es ist bekannt, Konsumenten durch die olfaktorische Wahrnehmung von Produkten anzusprechen. So werden zum einen öffentlich zugängliche Räume mit Geruchsaromen versetzt, um einen angenehmen Duft zu verbreiten oder auf spezielle Produkte aufmerksam zu machen. Zum anderen werden Verpackungen von Lebensmitteln mit Aromastoffen versetzt, so dass beim Öffnen der Verpackung ein angenehmer frischer Geruch verbreitet wird.

Bei all diesen Anwendungen ist es erforderlich, dass ein Trägergas mit einem Aroma versetzt wird. So ist zum einen bekannt, dass das Trägergas gemeinsam mit dem Aromagas gespeichert wird. Zum anderen ist bekannt, dem Trägergas das Aroma zuzudosieren, wenn das Aroma an dem gewünschten Ort eindosiert werden soll.

Aufgrund der Vielfalt an bekannten Aromen, die für diese Anwendungen zum Einsatz kommen können, ist es wünschenswert, dass ein Trägergas auf einfache Weise mit verschiedenen Aromen kombiniert werden kann.

Es ist daher Aufgabe der vorliegenden Erfindung, die mit Bezug auf den Stand der Technik geschilderten Probleme zumindest teilweise zu lösen. Insbesondere ist es Aufgabe der vorliegenden Erfindung, eine Vorrichtung und ein Verfahren anzugeben, mit denen bei einfacher Handhabung verschiedene Aromen in ein Trägergas eingespeist werden können.

Gelöst werden diese Aufgaben mit einer Vorrichtung und einem Verfahren gemäß den unabhängigen Ansprüchen. Vorteilhafte Weiterbildungen sind in den abhängigen Ansprüchen und in der Beschreibung angegeben.

Unter Aroma wird im Sinne der vorliegenden Erfindung wird nicht nur das Aroma selbst verstanden, sondern auch ein in einem Gas oder einer Flüssigkeit gelöstes Aroma, welches in Gasform in den Trägerstrom eingeleitet wird. Als Trägergas kommen alle für die verschiedenen Einsatzmöglichkeiten bekannten Gase und Mischungen mindestens zweier dieser Gase wie beispielsweise Druckluft in Betracht. Bevorzugt wird als Trägergas gasförmiger Stickstoff oder CO₂ (Kohlendioxid) eingesetzt.

Die Aromen können rein oder gemischt zugegeben werden. Die Zugabe kann in fester Form, als Flüssigkeit, als Gas, in chemisch gelöster, adoder absorbierter und/oder chemisch und/oder physikalisch gebundener Form zugegeben werden. Die Aromen können unter Druck, flüssig und/oder gasförmig in einer Ein- oder Wehrwegkartusche gespeichert werden. Insbesondere im Falle des Vorliegens als Gas können Trägergase zum Einsatz kommen, insbesondere Stickstoff, Helium, Xenon, Argon, Krypton, Wasserstoff, Kohlendioxid oder Mischungen mindestens zweier dieser Gase wie beispielsweise Druckluft zum Einsatz kommen.

Unter einem Anschluss wird ein Element verstanden, das eine mechanische Verbindung zwischen der Vorrichtung und dem Druckgasbehälter beziehungsweise der Aromaspeichereinheit herstellt und zum anderen eine strömungstechnische Verbindung der ersten Leitung und der zweiten Leitung mit dem Druckgasbehälter beziehungsweise mit der Aromaspeichereinheit gewährleistet. So ist in einer bevorzugten Ausführungsform der Anschluss für den Druckgasbehälter ein Innengewinde, das auf ein Außengewinde am Druckgasbehälter aufschraubbar ist und durch die im Betrieb zentral das Trägergas strömt. Als Auslass ist der Abschnitt einer Leitung aufzufassen, aus dem das Trägergas mit dem eingeleiteten Aroma aus der Vorrichtung austritt und zur weiteren Verwendung geleitet wird. So können die erste Leitung und die zweite Leitung gemeinsam in dem Anschluss enden, es ist aber bevorzugt, dass die erste Leitung und die zweite Leitung zusammengeführt werden und die so als dritte Leitung entstehende Leitung zum Auslass führt.

Die Aromaspeichereinheit umfasst bevorzugt einen Behälter, in dem das Aroma, gegebenenfalls in einer Lösung, gespeichert ist. Die Aromaspeichereinheit weist einen Schnellkupplungsanschluss auf, so dass der Behälter mit der zweiten Leitung strömungstechnisch verbunden werden kann.

Unter Schnellkupplungsanschlüssen werden Anschlussvorrichtungen verstanden, die ohne zusätzliches Werkzeug nur mit den Händen, bevorzugt nur mit einer Hand betätigt werden können. Der Schnellkupplungsanschluss an der Vorrichtung kann also insbesondere ein Stecker oder eine beziehungsweise eine Ausnahme sein, die ineinander gesteckt werden. Nach Zusammenbringen der Gegenstücke verrasten diese miteinander. Diese Verrastung kann mit einem einfachen Handgriff gelöst werden. Insbesondere erfolgt das Lösen der Rastverbindung durch eine Krafteinwirkung auf die Schnellkupplung in oder entgegen der Kupplungsrichtung. Eine Schnellkupplung erzeugt eine dem im Betrieb anliegenden Innendruck widerstehende mechanische Verbindung.

Aufgrund des Schnellkupplungsanschlusses ist es möglich, die Aromaspeichereinheit intuitiv, einfach und schnell auszutauschen. Dies erlaubt es zum einen, verschiedene Aromen an einem Einsatzort zu verwenden und zum anderen können so die Aromaspeichereinheiten für bestimmte Einsätze ausgelegt werden. So ist es bevorzugt, dass die Aromaspeichereinheiten wiederverwendbare Elemente aufweisen. Es ist aber auch möglich, dass die Aromaspeichereinheit als Wegwerfprodukt ausgelegt ist.

Gemäß der Erfindung ist die Vorrichtung unlösbar mit dem Druckgasbehälter verbunden. Unter unlösbarer Verbindung ist eine Verbindung zu verstehen, die nicht ohne Gewalteinwirkung und ohne Zerstörung der Vorrichtung getrennt werden kann. Durch die unlösbare Verbindung des Druckgasbehälters mit der Vorrichtung ist eine intuitive und einfache Bedienung gewährleistet. So ist das einzige Bauteil, was von der Vorrichtung getrennt werden kann, die Aromaspeichereinheit, die bei Bedarf ausgetauscht werden kann. Im Betrieb ist die Vorrichtung über den Auslass mit einer Leitung zu Zuführen des mit Aroma versetzten Trägergases zu einer Düse verbunden. Ist der Inhalt des Druckgasbehälters, also das Trägergas, verbraucht, so wird der Druckgasbehälter und die gesamte Vorrichtung ausgetauscht. Somit ist auch gewährleistet, dass die Vorrichtung regelmäßig, nämlich beim Austausch des Druckgasbehälters, gewartet und überprüft werden kann.

Gemäß noch einer weiteren vorteilhaften Ausgestaltung der Erfindung ist in der ersten Leitung und in der zweiten Leitung jeweils ein Absperrventil angeordnet, die bevorzugt über einen gemeinsamen Schalter gleichzeitig betätigbar sind. Durch das Betätigen des gemeinsamen Schalters können sowohl der Trägergasstrom als auch die Strömung aus der Aromaspeichereinheit gleichzeitig unterbrochen beziehungsweise wiederhergestellt werden, ohne dass direkt auf weitere Zustandsgrößen der Strömungen Einfluss genommen wird. So kann insbesondere ohne Veränderung des Mischungsverhältnisses von Trägerstrom und Aroma das Einleiten von Aroma in den Trägergasstrom begonnen beziehungsweise beendet werden.

Noch einer weiteren Weiterbildung der Vorrichtung folgend ist ein Druckreduzierventil oder ein Strömungsreduzierer zur schrittweisen Reduzierung der Strömung in vorgegebenen Schritten in der zweiten Leitung angeordnet. Durch das Druckreduzierventil kann die Strömung des Aromas und somit dessen Konzentration in der Mischung aus Trägergas und Aroma stufenlos eingestellt werden. Insbesondere ist das Druckreduzierventil unabhängig von dem Absperrventil. Dementgegen kann mit dem Strömungsreduzierer die Strömung schrittweise, beispielsweise in 2, 4, 6 oder 8 Schritten beeinflusst werden, wobei jedem Schritt eine gewisse Konzentration von Aroma in dem Trägergas zugeordnet werden kann. Auch diese schrittweise Reduzierung der Aromagasströmung findet unabhängig von dem Absperrventil statt.

Vorteilhaft ist zudem, wenn die Vorrichtung zumindest eines der folgenden Elemente umfasst:
- eine Füllstandsanzeige für die Druckgasbehälter und
- eine Schutzkappe, insbesondere mit einem Tragegriff.

Durch die Füllstandsanzeige ist auf einfache Weise zu ermitteln, wann der Druckgasbehälter leer sein wird und wann gegebenenfalls ein neuer Druckgasbehälter angebracht werden muss. Die Schutzkappe schützt die Vorrichtung vor mechanischen Krafteinwirkungen.

Es ist zudem vorteilhaft, wenn in der ersten Leitung und/oder in der zweiten Leitung ein Schalldämpfer integriert ist. Gerade an ruhigen Orten wird durch einen Schalldämpfer gewährleistet, dass die Vorrichtung im Betrieb keine Lärmbelästigung darstellt.

Gemäß einem weiteren Aspekt wird eine Aromaspeichereinheit zum Anschließen an eine erfindungsgemäße Vorrichtung vorgeschlagen, die einen Behälter, bevorzugt einen Druckgasbehälter, und zumindest eines der folgenden Elemente umfasst:
- ein Selbstabdichtelement,
- eine kalibrierte Öffnung,
- eine spannungsbasierte Füllstandsanzeige,
- einen Schnellkupplungsanschluss.

Mit der kalibrierten Öffnung wird gewährleistet, dass nach dem Austausch einer Aromaspeichereinheit bei gleichen Einstellungen an der Vorrichtung die gleiche Menge an Aroma in die Vorrichtung fließt wie vor dem Austausch. Der Schnellkupplungsanschluss garantiert eine Verbindung mit der erfindungsgemäßen Vorrichtung. Die auf einer Messung der mechanischen Spannung am äußeren Mantel des Behälters gemessene Spannung erlaubt auf einfache Weise, die Menge des verbleibenden Aromas in dem Behälter anzuzeigen. Das Selbstabdichtelement wiederum gewährleistet eine gasdichte Verbindung des Behälters über den Schnellkupplungsanschluss mit der erfindungsgemäßen Vorrichtung, in dem beim Schließvorgang der Schnellkupplung ein Dichtmaterial an eine entsprechende Dichtfläche gepresst wird. Ein Beispiel einer spannungsbasierten Füllstandsanzeige ist ein Dehnungsmessstreifen.

Einem weiteren Aspekt der Erfindung folgend wird ein Verfahren zum Einleiten eines Aromas in einen Trägergasstrom nach Anspruch 7 vorgeschlagen.

Die Einleitvorrichtung ist insbesondere eine erfindungsgemäße Vorrichtung. Somit ist aber gerade die erfindungsgemäße Vorrichtung zur Durchführung des erfindungsgemäßen Verfahrens geeignet. Die mit Bezug auf die erfindungsgemäße Vorrichtung offenbarten Merkmale und Vorteile lassen sich auf das erfindungsgemäße Verfahren übertragen und anwenden und umgekehrt.

Die Aromaspeichereinheit wird somit über den Schnellkupplungsanschluss mit der erfindungsgemäßen Vorrichtung verbunden und insbesondere anschließend wird der Trägergasstrom durch die Einleitvorrichtung geleitet, wobei gleichzeitig auch ein Einleiten des Aromas in die Einleitvorrichtung und somit in den Trägergasstrom erfolgt.

Es ist bevorzugt, dass der Aroma enthaltende Gasstrom in eine Verpackung für ein Lebensmittel eingeleitet wird. Insbesondere wird der Aroma enthaltende Gasstrom in eine Verpackung von Eis, Fleisch, Süßigkeiten, Snacks oder Getränken eingeleitet. Die Einleitung kann aber auch in einer Verpackung von Kosmetika erfolgen. Somit wird aber auch die Verwendung der erfindungsgemäßen Vorrichtung für das Einleiten von Aromagasen in entsprechende Verpackungen vorgeschlagen.

Alternativ ist auch bevorzugt, dass der Aroma enthaltende Gasstrom in einen vom Menschen benutzten Raum eingeleitet wird. Insbesondere kann das Aroma enthaltende Gas in ein Leihfahrzeug, ein öffentliches Verkehrsmittel, eine öffentliche Toilette, öffentliche Räume, Hotels, Lobby, Boutiquen, Bäckereien, Kaffeehäusern oder Flughäfen erfolgen. Somit wird aber auch gerade die Verwendung einer erfindungsgemäßen Vorrichtung zum Einleiten von aromatisierten Gasen in einen solchen von Menschen benutzten Raum vorgeschlagen.

Die Erfindung wird anhand der vorliegenden Zeichnung beispielhaft näher erläutert, ohne dass sie auf die dort gezeigten Ausführungsbeispiele beschränkt wäre.

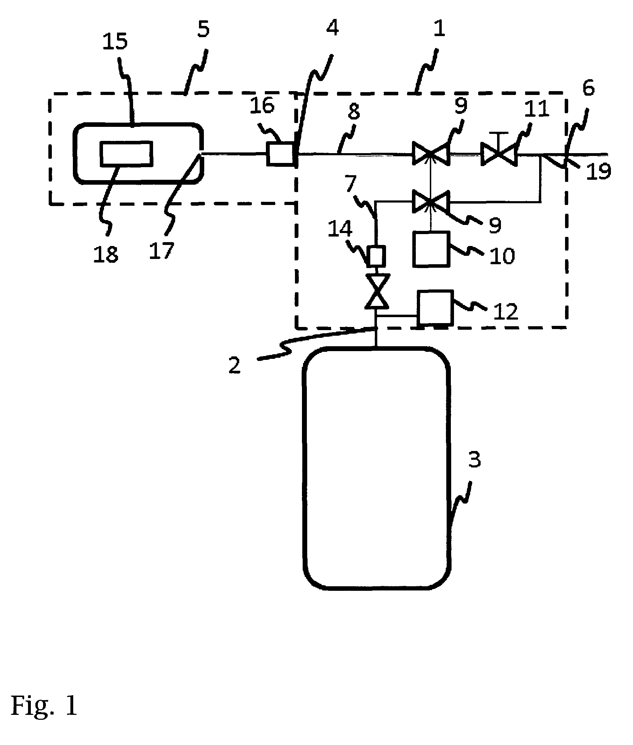

Die Fig. 1 zeigt schematisch eine Vorrichtung zum Einleiten eines Aromas in einen Trägergasstrom.

Die Fig. 1 zeigt eine Vorrichtung 1, an die ein Druckgasbehälter 3 und eine Aromaspeichereinheit 5 angeschlossen sind. Die Vorrichtung 1 weist einen ersten Anschluss 2 auf, über den die Vorrichtung 1 mit dem Druckgasbehälter 3 verbunden ist. In dem Druckgasbehälter 3 ist ein Trägergas gespeichert. Die Vorrichtung 1 weist ferner einen Schnellkupplungsanschluss 4 auf, über den die Vorrichtung 1 mit der Aromaspeichereinheit 5 verbunden ist. Mit dem ersten Anschluss 2 ist eine erste Leitung 7 strömungstechnisch verbunden und mit dem Schnellkupplungsanschluss 4 ist eine zweite Leitung 8 strömungstechnisch in der Vorrichtung 1 verbunden. Die erste Leitung 7 und die zweite Leitung 8 sind in der Vorrichtung 1 zusammengeführt und werden weiter als dritte Leitung 9 zu einem Auslass 6 geführt. Über den Auslass 6 ist die Vorrichtung 1 mit einer nicht dargestellten Einsprühdüse verbunden.

Sowohl in der ersten Leitung 7 als auch in der zweiten Leitung 8 ist jeweils ein Absperrventil 9 ausgebildet, das über einen gemeinsamen Schalter 10 betätigt werden kann. In der zweiten Leitung 8 ist ferner ein Druckreduzierventil 11 ausgebildet, das zur stufenlosen oder schrittweisen Reduzierung der Strömung in der zweiten Leitung 8 dient. In der ersten Leitung 7 ist ein Schalldämpfer ausgebildet, der eine mögliche Geräuschentwicklung beim Durchleiten des Trägergases durch die erste Leitung 7 mindert.

Die Aromaspeichereinheit 5 umfasst einen Behälter 15, der eine kalibrierte Öffnung 17 aufweist und einen spannungsinduzierten zweiten Füllstandsanzeiger 18. Zudem weist die Aromaspeichereinheit 5 einen Schnellkupplungsanschluss 4 auf, der mit einem Selbstdichtelement 16 versehen ist und über den die Aromaspeichereinheit 5 mit der Vorrichtung 1 mechanisch und strömungstechnisch verbunden wird.

Im Betrieb wird Trägergas aus dem Druckgasbehälter 3 über den ersten Anschluss 2 und durch die erste Leitung 7 zum Auslass 6 geleitet. Aromastoffe, die in gasförmiger Lösung oder flüssiger Lösung im Behälter 15 gespeichert sein können, werden über den Schnellkupplungsanschluss 4 und durch die zweite Leitung 8 in das Trägergas eingeleitet. Die Mischung aus Trägergas und Aroma gelangt zum Auslass, von wo das Gemisch zu einer geeigneten Düse für die jeweilige Anwendung geleitet wird. Über das stufenlose oder schrittweise einstellbare Druckreduzierventil 11 kann die Menge an zuzuleitendem Aroma eingestellt werden. Über den Schalter 10 können die beiden Absperrventile 9 betätigt werden, so dass gleichzeitig sowohl die Zuleitung von Trägergas aus dem Druckgasbehälter 3 als auch von Aroma aus dem Behälter 15 gestoppt beziehungsweise gestartet werden kann. Dabei muss insbesondere das Druckreduzierventil 11 nicht betätigt werden, so dass nach erneutem Beginn des Einleitens die gleichen Bedingungen vorliegen. Dadurch, dass ein Schnellkupplungsanschluss 4 zwischen Aromaspeichereinheit 5 und Vorrichtung 1 vorgesehen ist, kann bei verbrauchtem Aroma oder bei Aromawechsel ein einfacher Austausch der Aromaspeichereinheit 5 stattfinden. Zudem kann die Vorrichtung 1 unlösbar mit dem Druckgasbehälter 3 verbunden sein, so dass bei verbrauchtem Trägergas die komplette Einheit aus Vorrichtung 1 und Druckgasbehälter 3 ausgetauscht wird. Dadurch ist insbesondere gewährleistet, dass die Vorrichtung 1 regelmäßig gewartet wird.

Die vorliegende Erfindung ermöglicht die intuitive Bedienung einer Vorrichtung 1 zum Einleiten eines Aromas in ein Trägergas sowie das einfache Austauschen einer Aromaspeichereinheit 5.

### Bezugszeichenliste

- 1: Vorrichtung
- 2: erster Anschluss
- 3: Druckgasbehälter
- 4: Schnellkupplungsanschluss
- 5: Aromaspeichereinheit
- 6: Auslass
- 7: erste Leitung
- 8: zweite Leitung
- 9: Absperrventil
- 10: Schalter
- 11: Druckreduzierventil
- 12: erste Füllstandsanzeige
- 13: Schutzkappe
- 14: Schalldämpfer
- 15: Behälter
- 16: Selbsdichtelement
- 17: kalibrierte Öffnung
- 18: zweite Füllstandsanzeige
- 19: dritte Leitung

## Patentansprüche

1. Vorrichtung (1) zum Einleiten eines Aromas in einem Trägergasstrom, wobei die Vorrichtung (1) einen über einen ersten unlösbaren Anschluss (2) angeschlossenen Druckgasbehälter (3) aufweist, der das Trägergases speichert, und mindestens einen zweiten Anschluss (4) an einer Aromaspeichereinheit (5) und einen Auslass (6) für ein Gasgemisch, wobei der erste Anschluss (2) für den Druckgasbehälter (3) über eine erste Leitung (7) und der mindestens eine zweite Anschluss (4) für die Aromaspeichereinheit (5) über eine zweite Leitung (8) mit dem Auslass (6) strömungstechnisch verbunden sind, und wobei der zweite Anschluss (4) für die Aromaspeichereinheit (5) ein Schnellkupplungsanschluss (4) ist.

2. Vorrichtung (1) nach Anspruch 1, wobei in der ersten Leitung (7) und in der zweiten Leitung (8) jeweils ein Absperrventil (9) angeordnet ist, die über einen gemeinsamen Schalter (10) betätigbar sind.

3. Vorrichtung (1) nach einem der vorhergehenden Ansprüche, wobei ein Druckreduzierventil (11) zur stufenlosen oder schrittweisen Reduzierung der Strömung mit vorgegebenen Schritten in der zweiten Leitung (8) angeordnet ist.

4. Vorrichtung (1) nach einem der vorhergehenden Ansprüche, die zumindest eines der folgenden Elemente umfasst:
- eine erste Füllstandsanzeige (12) für den Druckgasbehälter (3) und
- eine Schutzkappe (13).

5. Vorrichtung (1) nach einem der vorhergehenden Ansprüche, wobei in der ersten Leitung (7) und/oder in der zweiten Leitung (8) ein Schalldämpfer (14) integriert ist.

6. Vorrichtung (1) nach einem der vorhergehenden Ansprüche, wobei die Aromaspeichereinheit (5) einen Behälter (15) und zumindest eines der folgenden Elemente umfasst:
- ein Selbstabdichtelement (16),
- eine kalibrierte Öffnung (17),
- eine spannungsbasierte zweite Füllstandsanzeige (18),
- einen Schnellkupplungsanschluss (4).

7. Verfahren zum Einleiten eines Aromas in einem Trägergasstrom, wobei das Aroma in einer Aromaspeichereinheit (5) gespeichert ist und das Trägergas in einem Druckgasbehälter (3) gespeichert ist, der unlösbar mit einer Einleitvorrichtung (1) verbunden ist, umfassend zumindest folgende Schritte:
- Verbinden der Aromaspeichereinheit (5) mit der Einleitvorrichtung (1) über einen Schnellkupplungsanschluss (4),
- Durchführen des Trägergasstroms aus dem Druckgasbehälter (3) durch die Einleitvorrichtung (1) zu einem Auslass (6),
- Einleiten des Aromas aus der Aromaspeichereinheit (5) in den Trägergasstrom in der Einleitvorrichtung (1).

8. Verfahren nach Anspruch 7, wobei der das Aroma enthaltende Gasstrom in eine Verpackung für ein Lebensmittel eingeleitet wird.

9. Verfahren nach Anspruch 7, wobei der das Aroma enthaltende Gasstrom in einen von Menschen benutzten Raum eingeleitet wird.

## Claims

1. Device (1) for introducing an aroma into a carrier gas stream, wherein the device (1) comprises a compressed gas container (3) connected via a first unreleasable connection (2), which compressed gas container (3) stores the carrier gas, and at least one second connection (4) to an aroma storage unit (5) and an outlet (6) for a gas mixture, wherein the first connection (2) for the compressed gas container (3) is connected via a first line (7) and the at least one second connection (4) for the aroma storage unit (5) is connected via a second line (8) to the outlet (6) by means of flow technology and wherein the second connection (4) for the aroma storage unit (5) is a quick-action coupling connection (4).

2. Device (1) according to claim 1, wherein in the first line (7) and in the second line (8) respectively a stop valve (9) is arranged which can be activated by a common switch (10).

3. Device (1) according to any one of the preceding claims, wherein a pressure-reducing valve (11) is provided for the stepless or stepped reduction of the flow with predefined steps in the second line (8).

4. Device (1) according to any one of the preceding claims which comprises at least one of the following elements:
- a first filling level display (12) for the compressed gas container (3) and
- a protective cap (13).

5. Device (1) according to any one of the preceding claims, wherein a silencer (14) is integrated into the first line (7) and/or into the second line (8).

6. Device (1) according to any one of the preceding claims, wherein the aroma storage unit (5) comprises a container (15) and at least one of the following elements:
- a self-sealing element (16),
- a calibrated opening (17),
- a voltage-based second filling level display (18),
- a quick-action coupling connection (4).

7. Method for introducing an aroma into a carrier gas stream, wherein the aroma is stored in an aroma storage unit (5) and the carrier gas is stored in a compressed gas container (3) which is connected unreleasably to an introduction device (1) comprising at least the following steps:
- connecting the aroma storage unit (5) to the introduction device (1) via a quick-action coupling connection (4),
- feeding the carrier gas stream from the compressed gas container (3) through the introduction device (1) to an outlet (6),
- introducing the aroma from the aroma storage unit (5) into the carrier gas stream in the introduction device (1).

8. Method according to claim 7, wherein the gas stream containing the aroma is introduced into food packaging.

9. Method according to claim 7, wherein the gas stream containing the aroma is introduced into a room used by people.

## Revendications

1. Dispositif (1) servant à introduire un arôme dans un flux de gaz porteur, dans lequel le dispositif (1) présente un contenant de gaz sous pression (3) raccordé par l'intermédiaire d'un premier raccord (2) inamovible, lequel stocke le gaz porteur, et au moins un deuxième raccord (4) à une unité de stockage d'arôme (5) et une sortie (6) pour un mélange de gaz, dans lequel le premier raccord (2) pour le contenant de gaz sous pression (3) est relié par l'intermédiaire d'un premier conduit (7) et l'au moins un deuxième raccord (4) pour l'unité de stockage d'arôme (5) est relié selon la technique des fluides par l'intermédiaire d'un deuxième conduit (8) à la sortie (6), et dans lequel le deuxième raccord (4) pour l'unité de stockage d'arôme (5) est un raccord de couplage rapide (4).

2. Dispositif (1) selon la revendication 1, dans lequel respectivement une soupape d'arrêt (9) est disposée dans le premier conduit (7) et dans le deuxième conduit (8), lesquelles soupapes d'arrêt peuvent être activées par l'intermédiaire d'un commutateur (10) commun.

3. Dispositif (1) selon l'une quelconque des revendications précédentes, dans lequel une soupape de réduction de pression (11) servant à réduire de manière continue ou de manière progressive l'écoulement à l'aide d'étapes prédéfinies est disposée dans le deuxième conduit (8).

4. Dispositif (1) selon l'une quelconque des revendications précédentes, lequel comprend au moins un des éléments suivants :
- un premier affichage de niveau de remplissage (12) pour le contenant de gaz sous pression (3), et
- un capuchon de protection (13).

5. Dispositif (1) selon l'une quelconque des revendications précédentes, dans lequel un silencieux (14) est intégré dans le premier conduit (7) et/ou dans le deuxième conduit (8).

6. Dispositif (1) selon l'une quelconque des revendications précédentes, dans lequel l'unité de stockage d'arôme (5) comprend un contenant (15) et au moins un des éléments suivants :
- un élément à étanchéification automatique (16),
- une ouverture (17) calibrée,
- un deuxième affichage de niveau de remplissage (18) basé sur la tension,
- un raccord de couplage rapide (4).

7. Procédé servant à introduire un arôme dans un flux de gaz porteur, dans lequel l'arôme est stocké dans une unité de stockage d'arôme (5) et le gaz porteur est stocké dans un contenant de gaz sous pression (3), qui est relié de manière inamovible à un dispositif d'introduction (1), comprenant au moins les étapes suivantes consistant à :
- relier l'unité de stockage d'arôme (5) au dispositif d'introduction (1) par l'intermédiaire d'un raccord de couplage rapide (4),
- faire passer le flux de gaz porteur provenant du contenant de gaz sous pression (3) en direction d'une sortie (6) en passant par le dispositif d'introduction (1),
- introduire l'arôme provenant de l'unité de stockage d'arôme (5) dans le flux de gaz porteur dans le dispositif d'introduction (1).

8. Procédé selon la revendication 7, dans lequel le flux de gaz contenant l'arôme est introduit dans un emballage pour un produit alimentaire.

9. Procédé selon la revendication 7, dans lequel le flux de gaz contenant l'arôme est introduit dans un espace utilisé par l'homme.
